# EUROPEAN PATENT APPLICATION

(11) **EP 2 769 736 A1**
(43) Date of publication of application: **27.08.2014**
(21) Application number: 13156318.1
(22) Date of filing: 22.02.2013
(51) Int. Cl.: A61K 39/395, A61K 31/375, A61P 25/24

(54) **Pharmaceutical composition for the treatment of burnout syndrome**

(71) Applicant: Bill, Anja, 97082 Würzburg (DE); Bill, Josip S., 97082 Würzburg (DE)
(72) Inventor: Bill, Josip S., 97082 Würzburg (DE)
(74) Representative: Isarpatent

(57) **Abstract**

The present invention is directed to a pharmaceutical composition or a kit-of-parts and its use in the treatment of burnout syndrome and longevity therapy.

## Description

The present invention is directed to a pharmaceutical composition or a kit-of-parts and its use in the treatment of burnout syndrome and longevity therapy.

### BACKGROUND OF THE INVENTION

Burnout is the psychological term for the experience of long-term exhaustion and diminished interest. Burnout is classified in Z73 of ICD-10 named "life-management difficulties". The European Agency for Safety and Health at Work estimates that the economic losses due to the burnout syndrome in the EU amount to approximately 22 billion Euro per year. Although there are attempts to prevent the burnout syndrome by appropriate measures focussing on work load, reward, control, community, fairness and values, the burnout syndrome seems to be relevant for an increasing number of working people.

Although no final figures are available it seems that burnout has become already a widespread disease. By results of a representative survey in 2011, physicians have diagnosed a burnout syndrome for 1.9 million people aged 14 to 65 years in Germany. Further, in the light of healthcare politics, 1.8 million sick leaves in 2010 due to a burnout are economically a huge burden.

Therapies, which are presently used for the treatment of a burnout are, among others, psychotherapy, especially cognitive behavioural therapy (CBT), phytotherapy, physiotherapy, adjuvant pharmacotherapy and complementary treatments like music therapy or body-mind therapies. The therapy of burnout depends mainly on the understanding of burnout, whether it is regarded as independent disease, as a preliminary stage of a depression or as a co-morbidity of depression.

The efficacy of therapies for the treatment of the burnout syndrome still is insufficiently investigated. Therefore, there is still a demand for medical therapies which address the physical and psychological needs of people suffering from burnout syndrome.

### DEFINITIONS

The term "composition" as used herein is intended to mean a composition for use in the administration to the human or animal body. Therefore, the composition according to the invention can be defined as a pharmaceutical or a therapeutic composition as well. Such a composition contains the active ingredients along with non-active components or excipients. The composition according to the invention can take the form of several types of dosage forms, including liquid dosage forms, solid dosage forms and semi-solid dosage forms which can also be differentiated by the method of administration.

A "kit-of-parts" according to the present invention means a composition of the active ingredients, further containing non-active ingredients, where the different components are not present in mixed, but spatially distinct form. An example of a kit-of-parts would be an assembly containing an injection solution comprising IgG antibodies and at least one component comprising a vitamin mixture which is, for example, in tablet or liquid form for oral administration.

According to standard definition, a composition comprises the different components in intimately mixed form, whereas a kit-of-parts contains the component in spatially distinct form.

### BRIEF DESCRIPTION OF THE INVENTION

A first aspect of the present invention relates to a composition or kit-of-parts comprising or consisting of
a) at least one component comprising a vitamin mixture; and
b) a component comprising IgG antibodies.

A second aspect of the present invention is related to such a composition or kit-of-parts for use in the treatment of burnout syndrome or in longevity therapy.

### DETAILED DESCRIPTION OF THE INVENTION

According to the first aspect of the invention, a composition or kit-of-parts comprises or consists of
a) at least one component comprising a vitamin mixture; and
b) a component comprising IgG antibodies.

The composition and the kit-of-parts will take the form of a pharmaceutical or therapeutic composition, i.e. will contain or consist of one or more active ingredients and one or more non-active ingredients or excipients.

According to the present invention, different options are available regarding the dosage form in which the composition or kit-of-parts according to the invention is present.

Component a) of the present composition or kit-of-parts, i.e. the at least one component comprising a vitamin mixture, might take different forms suitable for different ways of administration. Thus, the vitamin mixture may be present in a form suitable for peroral administration, i.e. in form of soft or hard shell capsules. Soft and hard shell capsules are widely used within the pharmaceutical and health food industry and have gained broad acceptance as they present pharmaceutical products in a form that is readily to be consumed and digested by a user. These capsules generally are made up of a shell and an active filling material. The shell is formed of readily digested materials, for example, a soft gelatine capsule which might comprise a mixture of gelatine, glycerol and water. Hard shell capsules generally comprise gelatine and water.

Water soluble vitamins such as B-group vitamins and ascorbic acid (vitamin C) are generally present in the form of a suspension in edible oil when encapsulated in a soft gelatine or hard shell capsules. For example, oils such as soya bean oil are generally used. Therefore, according to the present invention, component a) may be present in form of a soft or hard shell capsule comprising the mixture of the preferred vitamin ingredients.

Should soft shell capsules with fill-in liquids be used, the invention may be based on any liquid base system that can be encapsulated in a soft (or even hard) shell capsule. Examples of hydrophobic fill-in liquids include vegetable oil, vegetable oil derivatives or medium-chain triglycerides or mixtures thereof. Preferred examples of those vegetable oils include almond oil, arachis oil, borage oil, canola oil, fractionated coconut oil, lecithin, maize oil, olive oil, rapeseed oil, rice bran oil, soya bean oil, sunflower oil and many others.

Hydrophilic fill-in liquids include PEG's of different molecular weight (preferably from 300 to 8,000).

The soft or hard shell capsule may include any suitable amount of vitamin particles in suspension, but generally will include from 10 mg to 1,000 mg of vitamin as an active ingredient. The vitamin composition may include a mixture of coated vitamin particles or a combination of coated particles and other coated or uncoated active ingredients.

As an alternative, the vitamin component may take the form of a tablet or of a swallowable liquid such as a syrup or drops. In the latter case, the liquid might contain fruit flavours, odours and other additives to improve smell and taste of the liquid composition.

As a further alternative, component a) might take the dosage form of a parenteral composition, such as a solution for intravenous infusion or injection. Thus, the component advantageously may be provided as a formulation for infusion. This may be a pre-concentrate, to be diluted prior to administration, or a ready-to-use solution, fat emulsion or a dispersion. Regarding the scientific and legal requirements of manufacturing and administering parenteral solutions it is referred to Remington, The Science and Practice of Pharmacy, 22nd Edition, 2012. In particular, chapter 41, the formulation principles, vehicles and solutes as well as manufacturing processes gives a clear guidance on the manufacture of parenteral compositions.

The vitamins may be selected from the group of water soluble vitamins such as B- and C-group vitamins mentioned above or fat soluble vitamins such as vitamin H, vitamin A, vitamin D or vitamin E.

In a preferred embodiment, the vitamin mixture of the present invention comprises ascorbic acid, biotin, thiamine pyrophosphate, colecalciferol, cyanocobalamin, panthenol, folic acid, nicotinamide, pyridoxine hydrochloride, retinol palmitate, riboflavin-5'-phosphate and/or α-tocopherol. In a particularly preferred embodiment, the following vitamin mixture according to table 1 is used as part of an infusion solution of 500ml.

**Table 1**

| | |
|---|---|
| Ascorbic acid | 125 mg |
| Biotin | 69.00 µg |
| Thiamin | 3 mg |
| Colecalciferol | 5.0 µg |
| Cyanocobalamin | 6.0 µg |
| Dexpanthenol | 16 mg |
| Folic acid | 414.00 µg |
| Nicotinamide | 46.00 mg |
| Pyridoxine hydrochloride | 5 mg |
| Retinol palmitate | 1.0 mg |
| Riboflavin | 4.0 mg |
| α-Tocopherol | 10 mg |

Additionally, component a) may comprise minerals such as trace elements of iron, calcium, magnesium and zinc etc. in order to provide an additional benefit for the patient.

The mineral additives to be added to the vitamin mixture might be as follows (calculated based on an infusion of 500 ml):

**Table 2**

| | |
|---|---|
| K⁺ | 20.00 mmol |
| mg²⁺ | 5.00 mmol |
| Na⁺ | 20.00 mmol |
| Zn²⁺ | 12.00 µmol |
| Cu²⁺ | 6.00 µmol |
| Co²⁺ | 4.00 µmol |
| Mn²⁺ | 4.00 µmol |

The second component of the composition or kit-of-part according to the present invention comprises IgG antibodies. Immunglobulin G (IgG) is an antibody isotype. It is a protein complex composed of four peptide chains, namely two identical heavy chains and two identical light chains. There are four IgG subclasses in humans named in order of their abundance in serum (IgG1 being the most abundant): IgG1, IgG2, IgG3 and IgG4. Therefore, the IgG antibodies according to element b) of the present composition or kit-of-part usually will be a mixture of differing amounts of IgG1 to IgG4.

Preferably, the ratio between IgG1, 2, 3 and 4 used in component b) reflects the natural abundance of the IgG subclasses in serum. That is to say, IgG1 contributes to about 66%, IgG2 to about 23%, IgG3 to about 7% and IgG4 to about 4% to the overall amount of IgG antibodies. However, a certain degree of variation for each of the IgG subclasses is acceptable and equally effective. For example, the IgG composition might comprise 57% IgG1, 37% IgG2, 3% IgG3, 3% IgG4. Preferably, the IgG antibodies are human blood plasma IgG antibodies.

In a preferred embodiment, the kit-of-parts according to the present invention comprises or consists of at least two components, i.e. at least one component comprising a vitamin mixture and one component comprising IgG antibodies. As outlined above, the kit-of-parts in the present application means that the respective components are not present in mixed but in spatially distinct form. This allows a time-staggered application of the different components as will be outlined below.

The composition or kit-of-parts according to the present invention might be present in a suitable form for parenteral application, preferably in the form of a solution or suspension for injection or infusion.

In a second aspect, the composition or kit-of-parts of the present invention is claimed for use in the treatment of burnout syndrome or in longevity therapy. According to the data contained in the examples, the patients tested showed a quick response to the administered components already after the first day of treatment. Up to now, no side effects (or negative effects at all) have been shown.

In a preferred embodiment, the kit-of-parts of the present invention is used in the treatment of burnout syndrome or in longevity therapy, wherein the treatment/therapy comprises the following steps:
a) administering a component comprising a vitamin mixture;
b) administering a component comprising IgG antibodies; and
c) administering a further component comprising a vitamin mixture
to a patient in need thereof.

As an ideal setting for the present treatment, it turned out that steps a) to c) should be performed by intravenous infusion and should have a duration of approximately two hours each. Further, steps a) to c) preferably are repeated five times on five consecutive days followed by a break of one to two days, which in turn is followed by a five times repetition of steps a) to c) on five consecutive days.

Therefore, one single "treatment cycle" takes about five days which is interrupted by a break, where, optionally, a further treatment cycle is added after the break.

In a further preferred embodiment, the composition or kit-of-parts of the present invention is used in the treatment of burnout syndrome or the longevity therapy, where the patients treated belong to the elderly population.

In the following, the present invention is further illustrated by examples. They should in no case be interpreted as a limitation of the scope of the invention as defined in the claims.

### EXAMPLES

A group of patients according to Table 3 was subjected to the following anti-burnout and longevity treatment schedule:

Day 1 to 5:
- vitamin intravenous infusion of 500ml over 2 hours
- antibody intravenous infusion (human blood plasma IgG (57% IgG1, 37% IgG2, 3% IgG3, 3% IgG4)), 200ml over 2 hours
- vitamin intravenous infusion of 500ml over 2 hours
on each day.

The vitamin intravenous infusion was as defined in Table 1.

Following the first treatment cycle, a break of one to two days occurred followed by a second therapy cycle on therapy days six to ten:
- vitamin intravenous infusion of 500ml over 1hour
- antibody intravenous infusion (human blood plasma IgG (57% IgG1, 37% IgG2, 3% IgG3, 3% IgG4)), 200ml over 1 hour
- vitamin intravenous infusion of 500ml over 1 hour

The aim of the therapy was an IgG plasma level of a minimum of 4 to 6 g/l.

In Table 3, the results of an overall number of 20 patients along with their personal data (gender, age, pre-existing diseases, diagnosis etc.) are summarized.

**Table 3**

| No. | Abbr. | Gender | Age | Date of treatment | Pre exist. Disease | Diagnosis | Therapy | VAS prior to therapy | VAS after therapy | VAS 12 months later | Additional diseases ocurring within 24 months | Improvement of preexisting diseases within 24 months |
|---|---|---|---|---|---|---|---|---|---|---|---|---|
| 01 | G.B. | M | 55 | 7.-11.2.05 | HYP | BurnOut | Comb. th. | 2 | 10 | 9 | No | Yes |
| 02 | F.T. | M | 59 | 14.-18.2.05 | oB | BurnOut | Comb. th. | 2 | 10 | 10 | No | ./. |
| 03 | E.R. | M | 52 | 14.-18.2.05 | oB | BurnOut | Comb. th. | 2 | 10 | 10 | No | ./. |
| 04 | L.L. | M | 55 | 19.-23.2.05 | oB | BurnOut | Comb. th. | 1 | 10 | 10 | No | ./. |
| 05 | H.M. | M | 61 | 4.-8.4.05 | oB | BurnOut | Comb. th. | 1 | 10 | 10 | No | ./. |
| 06 | T.B. | M | 50 | 13.-17.6.05 | DM | BurnOut | Comb. th. | 2 | 10 | 10 | No | No |
| 07 | R.K. | M | 62 | 5.-9.9.05 | oB | BurnOut | Comb. th. | 2 | 10 | 9 | No | ./. |
| 08 | A.H. | M | 63 | 23.-27-1.06 | oB | BurnOut | Comb. th. | 1 | 10 | 10 | No | ./. |
| 09 | G.W. | M | 55 | 13.-17.3.06 | oB | BurnOut | Comb. th. | 2 | 10 | 10 | No | ./. |
| 10 | L.H. | M | 64 | 3.-7.7.06 | KHK | BurnOut | Comb. th. | 3 | 10 | 10 | No | No |
| 11 | A.G. | M | 60 | 10.-14.7.06 | oB | BurnOut | Comb. th. | 2 | 10 | 10 | No | ./. |
| 12 | T.Z. | M | 53 | 2.-6.10.06 | oB | BurnOut | Comb. th. | 1 | 10 | 9 | No | ./, |
| 13 | B.H. | F | 59 | 27.11.-1.12.06 | oB | BurnOut | Comb. th. | 1 | 10 | 10 | No | ./. |
| 14 | J.M. | M | 54 | 11.-15.12.06 | oB | BurnOut | Comb. th. | 1 | 10 | 10 | No | ./. |
| 15 | E.H. | M | 71 | 19.-23.2.07 | KHK | BurnOut | Comb. th. | 1 | 10 | 9 | No | No |
| 16 | J.G. | F | 59 | 26.2.-2.3.07 | HYP | BurnOut | Comb. th. | 1 | 10 | 10 | No | Yes |
| 17 | M.S. | M | 51 | 5.-9.3.07 | oB | BurnOut | Comb. th. | 2 | 10 | 10 | No | ./. |
| 18 | A.K. | M | 42 | 7.-11.5.07 | oB | BurnOut | Comb. th. | 1 | 10 | 10 | No | ./. |
| 19 | L.B. | M | 54 | 22.-26.10.07 | oB | BurnOut | Comb. th. | 1 | 10 | 10 | No | ./. |
| 20 | J.R. | M | 66 | 29.10.-2.11.07 | oB | BurnOut | Comb. th. | 1 | 10 | 10 | No | ./. |

| | | | | | | | | | | | | |
|---|---|---|---|---|---|---|---|---|---|---|---|---|
| Legend: oB = without medical finding HYP = hypertension KH K = coronary heart disease DM = diabetes mellitus Therapy: comb. th. = combination therapy (see above) VAS = visual analogue scale according to Hayes and Patterson (1921), scale 0-10: o points: worst impression of the general physical and mental performance 10 points: best individual impression of the general physical and mental performance | | | | | | | | | | | | |

From the above Table 3, it can be seen that the patients recovered rapidly and sustainably after receipt of the treatment of the invention. The average VAS value increased from 1.5 before therapy to 10 after therapy. Remarkably, the average VAS value still was 9.8 one year after the therapy.

Furthermore, no additional diseases occurred within 24 months after therapy. The two patients suffering from hypertension additionally showed an improvement of this pre-existing disease.

## Claims

1. A composition or kit-of-parts comprising or consisting of
a) at least one component comprising a vitamin mixture; and
b) a component comprising IgG antibodies.

2. The composition or kit-of-parts of claim 1, wherein the vitamin mixture comprises compounds selected from the vitamin groups of vitamin C, vitamin H, vitamin A, vitamin B, vitamin D and/or vitamin E.

3. The composition or kit-of-parts of claim 2, wherein the vitamin mixture comprises ascorbic acid, biotin, thiamine pyrophosphate, cholecalciferol, cyanocobalamin, panthenol, folic acid, nicotinamide, pyridoxine hydrochloride, retinol palmitate, riboflavin 5' phosphat, and/or alpha-tocopherol.

4. The composition or kit-of-parts of one or more of the preceding claims, wherein the IgG antibodies are human blood plasma IgG antibodies.

5. The kit-of-parts of one or more of the preceding claims, comprising or consisting of 2 components comprising a vitamin mixture and 1 component comprising IgG antibodies.

6. The composition or kit-of-parts of one or more of the preceding claims, which is in a suitable form for parenteral application, preferably in the form of a solution or suspension for injection or infusion.

7. The composition or kit-of-parts of one or more of the preceding claims for use in the treatment of burnout syndrome or in longevity therapy.

8. The kit-of-parts of one or more of the preceding claims for use in the treatment of burnout syndrome or in longevity therapy, wherein the treatment/therapy comprises the following steps:
a) administering a component comprising a vitamin mixture;
b) administering a component comprising IgG antibodies; and
c) administering a further component comprising a vitamin mixture to a patient in need thereof.

9. The kit-of-parts for use in the treatment of burnout syndrome or in longevity therapy of claim 8, where steps a)-c) are performed by intravenous infusion and have a duration of approximately 2 h each.

10. The kit-of-parts for use in the treatment of burnout syndrome or in longevity therapy of claims 8 or 9, wherein steps a)-c) are repeated 5 times on 5 consecutive days followed by a break of 1-2 days, which in turn is followed by a 5 times repeat of steps a)-c) on 5 consecutive days.

11. The composition or kit-of-parts of one or more of the preceding claims for use in the treatment of burnout syndrome or in longevity therapy, where the patients treated belong to the elderly population.
